**Europäisches Patentamt**

**European Patent Office**

**Office européen des brevets**

(19)

(11) Veröffentlichungsnummer : **0 344 158 B1**

(12) # EUROPÄISCHE PATENTSCHRIFT

(45) Veröffentlichungstag der Patentschrift :
**08.04.92 Patentblatt 92/15**

(51) Int. Cl.⁵ : **A61K 31/405, // (A61K31/405,
31:195, 31:405, 31:15)**

(21) Anmeldenummer : **88900071.7**

(22) Anmeldetag : **10.12.87**

(86) Internationale Anmeldenummer :
**PCT/EP87/00769**

(87) Internationale Veröffentlichungsnummer :
**WO 88/04170 16.06.88 Gazette 88/13**

(54) **SCHMERZPRÄPARAT.**

Die Akte enthält technische Angaben, die nach
dem Eingang der Anmeldung eingereicht
wurden und die nicht in dieser Patentschrift
enthalten sind.

(30) Priorität : **13.12.86 DE 3642668**

(43) Veröffentlichungstag der Anmeldung :
**06.12.89 Patentblatt 89/49**

(45) Bekanntmachung des Hinweises auf die
Patenterteilung :
**08.04.92 Patentblatt 92/15**

(84) Benannte Vertragsstaaten :
**AT BE CH DE FR GB IT LI LU NL SE**

(56) Entgegenhaltungen :
**EP-A-00 040 40
GB-A- 2 113 546
GB-A-21 135 45
US-A-46 394 65**

(56) Entgegenhaltungen :
**Unlisted Drugs, volume 25, No.9, September
1973, (Chatham, New Jersey, US), see page
144, Ref.k, "Madopar" cited in the application
Advances in Pain Research and Therapy,
vol.1, 1976, S.871-880**

(73) Patentinhaber : **KAMPRAD, Joachim
Kemperweg 67
W-4400 Münster (DE)**
Patentinhaber : **ROLF, Ludger
Hegerskamp 94
W-4400 Münster (DE)**

(72) Erfinder : **KAMPRAD, Joachim
Kemperweg 67
W-4400 Münster (DE)**
Erfinder : **ROLF, Ludger
Hegerskamp 94
W-4400 Münster (DE)**

(74) Vertreter : **von Kreisler, Alek, Dipl.-Chem. et al
Deichmannhaus am Hauptbahnhof
W-5000 Köln 1 (DE)**

EP 0 344 158 B1

**Beschreibung**

Die Erfindung betrifft die Verwendung von L-Tryptophan zusammen mit einem peripheren Abbauhemmer als Schmerzmittel mit verzögerter Wirkstoffreigabe.

Insgesamt stellt der Schmerz und dessen Bekämpfung eine der großen Herausforderungen an die heutige Medizin dar. Bedenkt man, daß der volkswirtschaftliche Schaden, der durch Schmerz entsteht, angesichts ca. 3 000 000 chronisch schmerzkranker Menschen allein in der Bundesrepublik Deutschland Milliarden beträgt, wird die gestellte Aufgabe besonders wichtig.

Bei der Behandlung chronisch schmerzkranker Menschen wird dieses Problem besonders deutlich. Zum einen ist die Einnahme der heute üblichen, analgetisch wirkenden Substanzen engen Grenzen bezüglich der Dauer und der Nebenwirkungen unterworfen, zum anderen wird das angestrebte Behandlungsziel in der Regel nicht oder nur unvollkommen erreicht. Der letzte Punkt führt häufig zu der im Prinzip unerlaubten Dosissteigerung, auch über die Höchstgrenze hinaus mit den damit verbundenen Nebenwirkungen, wie Blutungen, bösartige Tumore oder Leberbeschädigungen. Darüberhinaus bildet sich oft eine Medikamentenabhängigkeit und Sucht aus.

Die Erkenntnis, daß sich akute und chronische Schmerzen wesentlich voneinander unterscheiden, führt zu der Abkehr der meist nur unvollkommen wirkenden üblichen Analgetikatherapie. Hierbei wird über die Hemmung der Prostaglandinsynthese bei Läsionen im Gewebe die Aktivierung des nozizeptiven Schmerzsystems verhindert (Acetylsalicylsäure, Paracetamol, Metamizol).

Bei chronischen Schmerzsyndromen kommt es dagegen zu einer Verselbständigung des Schmerzes; eine Blockade der Prostaglandinsynthese wird damit sinnlos. Dieses führt zu dem Einsatz thymoleptisch bzw. neuroleptisch sowie zentral wirkender Arzneimittel (Psychopharmaka, Opiate u.a.). In diesem Fall versucht man höhere Zentren des Schmerzsystems zu erreichen und zu beeinflussen. Ein gutes Beispiel ist der Phantomschmerz (Schmerzen in einem nicht mehr vorhandenen Körperteil) oder die veränderte Schmerzschwelle bei Depressionen. Die dabei auftretenden erheblichen Nebenwirkungen als auch die geringe Wirkung dieser Medikamentengruppe setzte auch hier der Therapie enge Grenzen.

Im Zuge der modernen Schmerzforschung wurden die Strukturen schmerzleitender und schmerzverarbeitender Systeme auf allen Ebenen immer durchsichtiger und begreifbarer. Hierbei entdeckte man neben den Systemen der Schmerzwahrnehmung auch Systeme der Schmerzkontrolle, welche mittels chemischer Botenstoffe (Neurotransmitter) auf die Weiterleitung von Schmerzimpulsen einwirken. Einer der am besten untersuchten Neurotransmitter mit seinen dazugehörigen neuronalen Systemen stellt das Serotonin dar. Dieses wirkt auf allen neuronalen Ebenen, indem es in Verbindung mit dem Opioid-System bevorzugt die Schmerzweiterleitung hemmt und die Schmerzschwelle anhebt. Aus diesem Grunde wäre das Serotonin eine ideale physiologische Substanz zur Inhibierung von Schmerzreizen, Veränderung der Schmerzverarbeitung und der Schmerzkontrolle (Limbisches System und Hirnstamm). Da man Serotonin jedoch wegen erheblicher Nebenwirkungen nicht direkt dem Menschen zuführen kann, benutzt man allgemein ein Prinzip, welches auch bei anderen Neurotransmittern angewendet wird. Hierbei gibt man anstelle des Neurotransmitters in hoher Konzentration eine biochemische Vorstufe (Präkursor) desselben.

Als Präkursor verwendet man in der Regel Naturstoffe oder Nahrungsbestandteile. Am Beispiel des Parkinsonismus erläutert, gibt man als Präkursor L-DOPA, um einen systemischen Mangel von Dopamin im Gehirn auszugleichen. Allerdings werden die üblicherweise verwendeten Präkursoren bereits peripher in hohem Maße, d.h. im Blut sowie im Magen-Darm-Trakt, zu den im Prinzip erwünschten Substanzen abgebaut. Da die Neurotransmitter als solche nicht Blut-Hirnschranken gängig sind, überschwemmen sie die Peripherie, treten aber praktisch nicht in das Gehirn über. Das hat die bekannten peripheren Nebenwirkungen zur Folge, wie Übelkeit, Erbrechen, Herz-Kreislaufstörungen, Blutdruckänderungen usw. Um diese Nebeneffekte zu vermeiden, kombiniert man L-DOPA mit peripheren Abbauhemmern. Als Folge davon reichert sich das L-DOPA im Blut an und kann in ausreichend hoher Menge die Blut-Hirnschranken überwinden; dort wird L-DOPA wie erwünscht zum Dopamin abgebaut.

Auch bei der Indikation "Schmerz" experimentiert man bis heute mit Präkursoren. Hierbei gibt man den Nahrungsmittelbestandteil Tryptophan im Überschuß, um auf biologisch-physiologische Weise die Bildung der "Anti-schmerzsubstanz" Serotonin zu steigern.

Durch den Abbau des Tryptophans in der Peripherie kommt es jedoch zur Anreicherung des Serotonins am falschen Ort und damit zu unerwünschten Nebenwirkungen, wie Blutdruckkrisen, chronische Diarrhoe, Bronchospasmus, kardiale Störungen, Magen- und Darmstörungen u.a.. Nur eine gernige Menge L-Tryptophan entgeht dem peripheren Abbau und kann ungehindert in das Zentralnervensystem gelangen und dort zum gewünschten Transmitter abgebaut werden. Versuche, möglichst große Mengen L-Tryptophan zu geben, um eine wirksame Anreicherung dieser Aminosäure zu erzielen, scheiterten an den dann auftretenden Nebenwirkungen.

Aus diesem Grunde hat dieser Naturstoff bis heute keine praktische Bedeutung in der Schmerzbekämpfung erlangt. Damit scheiterte der Einsatz einer biologisch-physiologisch wirkenden Substanz in der Schmerztherapie. Stattdessen setzte man auf das unphysiologische Prinzip einer Manipulation des schmerzhemmenden Systems mit Hilfe von Psychopharmaka und zentral wirkenden Analgetika (Opiate oder dergleichen).

Tryptophan ist in den meisten Proteinen zu 1 - 2 % enthalten. Damit ist diese Substanz ein Naturstoff, der in der üblichen Nahrung des Menschen vorkommt. Insgesamt gibt es vier Abbauwege des Tryptophan. Einer dieser Abbauwege im menschlichen Körper führt über das 5-Hydroxytryptophan (5-HTP) nach Decarboxylierung zum 5-Hydroxytryptamin (5-HT = Serotonin). Serotonin ist in der Natur weit verbreitet und wird beim Säugetier in relativ hoher Konzentration im Zentralnervensystem (Hypothalamus, Periaquäduktales Grau, Zentrales Höhlengrau, Limbisches System), in der Milz, der Lunge und in den argentaffinen Zellen des Darmtraktes gefunden. Die Konzentration im Vollblut beträgt 0,1 - 0,3 µg/ml.

Serotonin hat peripher Wirkung auf die glatte Muskulatur der Gefäße, des Respirations- und des Gastrointestinaltraktes u.a. Eine besonders bedeutungsvolle Wirkung übt das Serotonin auf das zentrale Nervensystem aus. Eine dieser Wirkungen bezieht sich auf die Schmerzregulation.

Versuche, L-Tryptophan als wirksames Schmerzmittel einzusetzen, sind bisher gescheitert. Nach S. Seltzer et al (1982) vermag L-Tryptophan die Schmerzschwelle nicht zu beeinflusssen (Doppelblindversuch), jedoch ist es in der Lage, die Schmerztoleranzgrenze, d.h. die Grenze, ab welcher der Schmerz unerträglich wird, in gewissem Umfang (12 %) zu verbessern. Eine Verbesserung der Schmerzschwelle, das bedeutet eine Erhöhung der Schwelle, ab welcher ein Reiz als Schmerzreiz empfunden wird, läßt sich unter normalen Umständen nicht mit L-Tryptophan erreichen.

Die Verwendung der Kombination von L-DOPA und dem spezifischen Decarboxylasehemmer Benserazid nebst einem Hydrocolloid und einigen konventionellen Hilfsstoffen als Präparat mit verzögerter Wirkstoff-Freisetzung wird in der DE-OS 32 32 873 beschrieben.

Die Verwendung von L-Tryptophan allein zur Behandlung chronischer Schlafstörungen oder depressiven Zuständen ist in den L-Tryptophan A.S.-Tabletten (Fa. A.S. Biologische und pharmazeutische Produkte GmbH), Tryptocompren[R] (Fa. Cascan) und Kalma[R] Tabletten (Fa. Fresenius) verwirklicht.

Die Verwendung von L-Tryptophan oder L-5-Hydroxytryptophan in Verbindung mit Benserazid und die damit verbundenen geringen Dosen von L-Tryptophan bzw. L-5-Hydroxytryptophan werden von F. Sicuteri beschrieben in "Advances in Pain Research and Therapy" Vol. 1, 1976.

Schlaf stellt eine lebensnotwendige Verminderung der Bewußtseinslage dar, bei der es in fast allen Organen zu Regenerations- und Aufbauprozessen kommt. Hierbei besteht ein prinzipieller Unterschied zwischen Schlaf, Bewußtlosigkeit und Narkose. Während der Schlaf ein physiologischer und reversibler Vorgang ist, sind Bewußtlosigkeit und Narkose unphysiologische, nicht jeder Zeit reversible Prozesse.

Schmerz dagegen stellt eine ungenehme Sinneswahrnehmung und ein emotionales Gemütsleben dar, das mit Gewebsläsionen assoziiert ist, bzw. mit Empfindungen wie bei Gewebsläsionen beschrieben wird (Amerikanische Gesellschaft für Schmerzforschung). Dem Schmerz kommt dabei eine physiologische Schutzfunktion zu, um als Signal auf Verletzungsgefahren und Belastungssituationen notwendige Reaktionen zu provozieren.

Zur Behandlung chronischer Schmerzen beim Menschen wurde L-Tryptophan allein bisher gelegentlich klinisch erprobt. Bei der Verwendung von L-Tryptophan allein müssen wenigstens ca. 3 g oral angewandt werden, damit eine analgetische Wirkung auftritt. Das hängt damit zusammen, daß ein großer Teil des verabreichten L-Tryptophans peripher, beispielsweise durch das Enzym aromatische Aminosäuredecarboxylase oder andere Enzyme, abgebaut wird, bevor es die Bluthirnschranke passieren kann, und es nach Umwandlung zu Serotonin biologisch wirksam wird. Durch eine Blockade der peripheren aromatischen Aminosäuredecarboxylase und Kynureninase wird die Menge an L-Tryptophan, welche die Blut-Hirnschranke passiert, beträchtlich erhöht.

Der vorliegenden Erfindung liegt die Aufgabe zugrunde, ein neuartiges Schmerzmittel zur Verfügung zu stellen, das durch die Verwendung von L-Tryptophan zusammen mit einem peripheren Abbauhemmer erhalten-wird.

Weitere Aufgabe der vorliegenden Erfindung ist ein pharmazeutisches Präparat mit verzögerter Wirkstofffreigabe, das L-Tryptophan und insbesondere einen peripheren Decarboxylasehemmer enthält, das überlegene Blutspiegel von L-Tryptophan bewirkt und dadurch bei chronischem Schmerz für Menschen ein physiologisches Schmerzmittel mit zentraler Wirkung darstellt.

Die vorstehend genannten Aufgaben werden durch die Verwendung von L-Tryptophan und einem peripheren Abbauhemmer für L-Tryptophan zur Herstellung eines Schmerzmittels mit verzögerter Wirkstofffreigabe für Menschen gelöst, wobei das Gewichtsverhältnis von L-Tryptophan zu peripherem Abbauhemmer 3 : 1 bis 10 : 1 beträgt.

Eine Verbesserung des Transportes von L-Tryptophan vom Blut ins Gehirn ist möglich durch Anwendung von peripheren Abbauhemmern gemeinsam mit L-Tryptophan.

L-Tryptophan (Indolyl-3-alanin) stellt eine physiologische Verbindung (essentielle Aminosäure) dar, die zur Behandlung von Schlaflosigkeit, depressiven Syndromen (endogene Depressionen) und psychotischer Nebenwirkungen der L-DOPA Therapie des Parkinsonismus eingesetzt wird.

Auch periphere Abbauhemmer, d.h. insbesondere Decarboxylasehemmer sind klinisch verwendete Substanzen, z.B. Benserazid und Carbidopa in den Parkinsonmitteln Madopar bzw. Nacom.

Gemäß einer bevorzugten Ausführungsform der vorliegenden Erfindung wird ein Gewichtsverhältnis von L-Tryptophan zu peripherem Abbauhemmer von 3 : 1 bis 5 : 1 eingesetzt.

Vorzugsweise werden gemäß einer weiteren Ausführungsform der vorliegenden Erfindung als periphäre Abbauhemmer für L-Tryptophan periphäre Decarboxylasehemmer und/oder Kynureninasehemmer eingesetzt.

Geeignete Decarboxylasehemmer im Sinne der vorliegenden Erfindung sind α-Methyldopa, m-Hydroxybenzylhydrazin, L-α-Hydrazino-3,4-dihydroxy-α-methyl-hydrozimtsäure (Carbidopa) und N$^1$-DL-Serin-N$^2$-(2,3,4-trihydroxybenzyl)-hydrazid-hydrochlorid (Benserazid).

Der Serotoningehalt menschlicher Thrombozyten, welche ein Modell zentraler serotoninerger Neurone darstellen, ist bei Anwendung des erfindungsgemäßen Präparates höher als dies bei Präparaten mit gleichem L-Tryptophangehalt ohne Benserazid beobachtet wurde. Darüber hinaus verzögert das erfindungsgemäße Präparat ein Absinken der Serotoninwerte der Thrombozyten, verglichen mit L-Tryptophanpräparaten ohne Decarboxylasehemmer.

Aus den US-Patentschriften 4 126 672, 4 140 755, 4 167 558 und der DE-OS 32 32 873 sind Formulierungen mit verzögerter Wirkstofffreigabe bei oraler Applikation bekannt. Es handelt sich um Kapseln bzw. Tabletten, die hydrodynamisch so ausgewogen sind, daß sie ein spezifisches Gewicht von unter 1 besitzen und im Magensaft mit einem spezifischen Gewicht von 1,004 bis 1,010 aufschwimmen. Die verzögerte Wirkstofffreigabe dieser Formulierungen beruht auf einem Gemisch der aktiven Wirkstoffe und einem oder mehreren hydrophilen Hydrocolloiden.

Decarboxylasehemmer wirken durch Inhibierung des Enzyms aromatische Aminosäuredecarboxylase. Die verwendeten peripheren Decarboxylasehemmer Benserazid und Carbidopa sind darüberhinaus auch Hemmer der Kynureninase sowie der 2,3 Dioxygenase. Das führt bei gleichzeitiger Gabe von L-Tryptophan zu erhöhten L-Tryptophan-Werten im Plasma.

Die Herstellung der Präparate erfolgt nach allgemein bekannten Verfahren. Sie bestehen in einer gründlichen Durchmischung aller Bestandteile zu einem homogenen Gemisch und Vermahlung bzw. Zerkleinerung des Gemisches auf eine relativ kleine Partikelgröße (z. B. 100 mesh Siebweite).

Die Herstellung der Tabletten erfolgt vorzugsweise so, daß die Inhaltsstoffe zunächst granuliert und anschließend mit Tablettiermaschinen zu Tabletten gepreßt werden.

Im folgenden sollen zur Erläuterung der obigen Ausführungen einige Formulierungsbeispiele aufgeführt werden:

Beispiel 1

Formulierung I

Kapsel mit verzögerter Wirkstofffreigabe folgender Zusammensetzung:

| | |
|---|---|
| L-Tryptophan | 104,40 mg/Kapsel |
| Benserazid | 26,30 mg/Kapsel |
| Monocalciumphosphat | 14,80 mg/Kapsel |
| Hydriertes Baumwollsamenöl | 30,20 mg/Kapsel |
| Hydroxypropylcellulose | 4,10 mg/Kapsel |
| Hydroxypropylmethylcellulose | 114,90 mg/Kapsel |
| Mannit | 20,10 mg/Kapsel |
| Talk | 14,80 mg/Kapsel |
| Gesamt | 339,00 mg/Kapsel |

L-Tryptophan, Benserazid, Monocalciumphosphat und hydriertes Baumwollsamenöl wurden in den oben angegebenen Mengen miteinander vermischt und gemahlen. Anschließend erfolgte die Granulierung dieser Pulvermischung mit Hydroxypropylcellulose, welche zuvor in Alkohol gelöst worden war. Darauf wurde ein gemahlenes Gemisch aus Hydroxypropylmethylcellulose und Mannit zum Granulat gegeben. Das erweiterte Gemisch wurde einem Trockenvorgang unterworfen, mit Talk gemischt und in Kapseln abgefüllt.

Formulierung II

Tabletten mit verzögerter Wirkstofffreigabe folgender Zusammensetzung:

| | |
|---|---|
| L-Tryptophan | 187,50 mg/Tablette |
| Calciumcarbonat | 45,80 mg/Tablette |
| Carboxymethylcellulose | 45,70 mg/Tablette |
| Mannit | 21,10 mg/Tablette |
| Polyvinylpyrrolidon | 9,20 mg/Tablette |
| Hydroxypropylmethylcellulose | 100,80 mg/Tablette |
| Benserazid | 53,20 mg/Tablette |
| Fumarsäure | 22,90 mg/Tablette |
| Talk | 11,90 mg/Tablette |
| Magnesiumstearat | 2,80 mg/Tablette |
| Gesamt | 500,90 mg/Tablette |

Es wurde unter Verwendung von Alkohol und einem Teil des Polyvinylpyrrolidons ein Granulat aus L-Tryptophan, Calciumcarbonat, Carboxymethylcellulose und Mannit hergestellt. Anschließend wurde dem Granulat Hydroxypropylmethylcellulose zugemischt und das Gemisch über Nacht getrocknet. Benserazid und Fumarsäure wurden gemischt und mit dem restlichen Polyvinylpyrrolidon ebenfalls in Alkohol granuliert und getrocknet. Beide Granulate wurden mit Talk und Magnesiumstearat gemischt und zu Tabletten mit einer Härte von 5 - 8 Strong-Cobb-Einheiten verarbeitet. Die Härte von 12 Strong-Cobb-Einheiten wurde nicht überschritten.

Beispiel 2

In vivo Untersuchungen

Die Präparate von Beispiel 1 wurden auf das Verhalten der Thrombozyten gesunder Probanden gegenüber Serotonin untersucht. Wie bereits geschildert, ist nicht L-Tryptophan der physiologische Wirkstoff im menschlichen Organismus, sondern das aus ihm in zentralen serotoninergen Neuronen gebildete Serotonin. Dieses erhöht im Gehirn die bei chronischem Schmerz gesenke Schmerzschwelle. Anerkanntes Modell für zentrale serotoninerge Neurone stellen die Blutplättchen dar. Es gilt die Regel, daß Manipulationen, welche den Serotoningehalt in den Blutplättchen erhöhen, auch den Serotoningehalt in zentralen serotoninergen Neuronen steigern und daß die Erhöhung des Blutplättchen-5-HT-Gehaltes mit vermindertem zentralbedingten Schmerz korreliert.

Die Probanden nahmen umgerechnet 1 g L-Tryptophan und 250 mg Benserazid der Präparate in Beispiel 1 in einer einmaligen Dosis morgens oral zu sich. Vor der Medikamenteneinnahme und in einem halbstündlichen Abstand bis zu 6 Stunden danach wurde den Probanden Blut entnommen und der Blutplättchen-Serotoningehalt der einzelnen Proben spektralfluorometrisch bestimmt. In einem zweiten Versuch, der 3 Wochen nach dem oben beschriebenen Versuch durchgeführt wurde, erhielten dieselben Versuchspersonen 1 g L-Tryptophan ohne Benserazid. In beiden Zeitversuchen stieg der Blutplättchen-Serotoningehalt unter der L-Tryptophanbelastung jeweils an, jedoch lag der Serotoningehalt im Versuch mit Benserazid im Durchschnitt um etwa 40 % höher als im Versuch ohne Benserazid. Darüber hinaus war der Effekt im Versuch mit Benserazid noch über 4 Stunden nach Versuchsbeginn, im Versuch ohne Benserazid dagegen nur bis 2 Stunden nach Versuchsbeginn nachweisbar.

**Patentansprüche**

1. Verwendung von L-Tryptophan und einem periphären Abbauhemmer für L-Tryptophan zur Herstellung eines Schmerzmittels mit verzögerter Wirkstoffreigabe für Menschen, wobei das Gewichtsverhältnis von L-Tryptophan zum peripheren Abbauhemmer 3 : 1 bis 10 : 1 beträgt.

2. Verwendung nach Anspruch 1, dadurch gekennzeichnet, daß das Gewichtsverhältnis von L-Tryptophan zu peripherem Abbauhemmer 3 : 1 bis 5 : 1 beträgt.

3. Verwendung nach den Ansprüchen 1 und 2, dadurch gekennzeichnet, daß man als peripheren Abbauhemmer für L-Tryptophan einen peripheren Decarboxylasehemmer und/oder Kynureninasehemmer einsetzt.

4. Verwendung nach Anspruch 3, dadurch gekennzeichnet, daß der periphäre Decarboxylasehemmer ausgewählt ist aus $\alpha$-Methyldopa, m-Hydroxybenzylhydrazin, $N^1$-DL-Serin-$N^2$-(2,3,4-trihydroxybenzyl)-hydrazid, L-$\alpha$-Hydrazino-3,4-dihydroxy-$\alpha$-methyl-hydrozimtsäure sowie deren Gemische.

5. Verwendung nach einender Ansprüche 1 bis 4, wobei das Präparat 5 bis 80 Gew.-% eines Hydrocolloids oder eines Gemisches von Hydrocolloiden, bis 60 Gew.-% eines Feststoffes oder eines Gemisches von Feststoffen und bis zu 80 Gew.-% von inerten, eßbaren pharmazeutischen Hilfsstoffen enthält und das Präparat in Kapsel- oder Tablettenform hydrodynamisch ausgewogen ist, so daß es in Kontakt mit Magenflüssigkeit insgesamt ein spezifisches Gewicht von unter 1 annimmt, so daß es in der Magenflüssigkeit aufschwimmt.

**Claims**

1. Use of L-tryptophane and a peripheral degradation inhibitor for L-tryptophan for the preparation of sustained release analgesic drug for humans, the ratio by weight of L-tryptophan to the peripheral degradation inhibitor being from 3:1 to 10:1.

2. The use according to claim 1, characterized in that the ratio by weight of L-tryptophan to the peripheral degradation inhibitor is from 3:1 to 5:1.

3. The use according to claims 1 and 2, characterized in that a peripheral decarboxylase inhibitor and/or a kynureninase inhibitor is used as the peripheral degradation inhibitor for L-tryptophan.

4. The use according to claim 3, characterized in that the peripheral decarboxylase inhibitor has been selected from $\alpha$-methyldopa, m-hydroxybenzylhydrazine, $N^1$-D,L-serine-$N^2$-(2,3,4-trihydroxybenzyl)-hydrazide, L-$\alpha$-hydrazino-3,4-dihydroxy-$\alpha$-methylhydrocinnamic acid and mixtures thereof.

5. The use according to anyone of claims 1 to 4, wherein the formulation contains from 5 to 80% by weight of a hydrocolloid or a mixture of hydrocolloids, up to 60% by weight of a solid or a mixture of solids, and up to 80% by weight of inert edible pharmaceutical auxiliary materials and the formulation in the form of capsules or tablets is hydrodynamically balanced so that upon contact with the gastric fluid altogether it will assume a specific weight of less than 1 so that it will float on the gastric fluid.

**Revendications**

1. Utilisation du L-tryptophanne et d'un inhibiteur périphérique de la dégradation du L-tryptophanne pour produire un analgésique , à libération retardée de sa substance active, pour des êtres humains, dans laquelle le rapport pondéral du L-tryptophanne à l'inhibiteur périphérique de dégradation est de 3:1 à 10:1 .

2. Utilisation selon la revendication 1 , caractérisée en ce que le rapport pondéral entre le L-tryptophanne et l'inhibiteur périphérique de dégradation est de 3:1 à 5:1 .

3. Utilisation selon les revendications 1 et 2 , caractérisée en ce qu'on utilise comme inhibiteur périphérique de la dégradation du L-tryptophanne un inhibiteur de décarboxylase périphérique et/ou un inhibiteur de kynuréninase .

4. Utilisation selon la revendication 3 , caractérisée en ce que l'inhibiteur de décarboxylase périphérique est choisi parmi l'$\alpha$-méthyldopa , la m-hydroxybenzylhydrazine , le $N^1$- DL-sérine-$N^2$-(2,3,4-trihydroxybenzyl)hydrazide ,l'acide L-$\alpha$-hydrazino-3,4-dihydroxy- $\alpha$-méthyl-hydrocinnamique ainsi que parmi leurs mélanges.

5. Utilisation selon une des revendications 1 à 4 , dans laquelle la préparation convient 5 à 80 % en poids d'un hydrocolloïde ou d'un mélange d'hydrocolloides , jusqu'à 60 % en poids d'un solide ou d'un mélange de solides et jusqu'à 80% en poids d'adjuvants pharmaceutiques comestibles inertes et en ce que la préparation est pesée sous forme de capsules,gélules ou comprimés de manière à présenter un équilibrage hydrodynamique tel qu'au contact du suc gastrique elle prenne une densité inférieure à 1 de manière à flotter ou surnager dans le suc gastrique .